# EUROPEAN PATENT SPECIFICATION

(11) **EP 2 753 269 B1**
(45) Date of publication and mention of the grant of the patent: **05.10.2016**
(21) Application number: 12769498.2
(22) Date of filing: 05.09.2012
(51) Int. Cl.: A61F 2/30, A61F 2/40

(54) **SHOULDER JOINT PROSTHESIS**
SCHULTERGELENKPROTHESE
PROTHÈSE D'ARTICULATION D'ÉPAULE

(30) Priority: 06.09.2011 FR 1157872
(43) Date of publication of application: 16.07.2014
(73) Proprietor: 3S Ortho, 69009 Lyon (FR)
(72) Inventor: NOVE-JOSSERAND, Laurent, 69300 Caluire (FR); COURJAUD, Xavier, 17110 Saint Georges De Didonne (FR); MILET, Alexandre, 72000 Le Mans (FR)
(74) Representative: Jeannet, Olivier
(86) International application number: PCT/IB2012/054566
(87) International publication number: WO 2013/035039

(56) References cited:
- EP-A1- 0 953 321
- EP-A1- 1 611 872
- EP-A2- 1 285 639
- WO-A2-03/053280
- FR-A1- 2 763 501

## Description

The present invention relates to a shoulder joint prosthesis. It also relates to a set of configurable elements making it possible to form that prosthesis. The features of the preamble of claim 1 are known from EP-A-0953321.

It is well known to repair a fractured shoulder joint using a shoulder prosthesis including a humeral shaft inserted into the medullary conduit of the humerus, a proximal portion mounted on that shaft, forming a articular surface, and a scapula implant, anchored to the scapula and forming a articular surface complementary to the articular surface formed by said proximal portion.

On a shoulder prosthesis, it is particularly important for the proper operation of the restored joint for the concerned bones to be re-situated in anatomical positions, so as to obtain good ligament tensions and good balance of the ligament tensions. Failing this, the operation of the prosthetic joint is affected.

The existing prostheses are not fully satisfactory from this perspective, and one primary aim of the present invention is to resolve this drawback.

Furthermore, the placement of a shoulder prosthesis frequently involves repositioning bone fragments of the humerus, in particular tuberosities, which are frequently fractured. This placement involves ligating those tuberosities to the humeral shaft, using suture threads. To that end, the existing prostheses generally comprise a fin on the outer side, passed through by holes for the engagement of said suture threads.

Such an external fin is not fully satisfactory in terms of wedging of the humeral shaft in the humerus, and the placement of the threads through the holes of that fin is relatively difficult in practice, given that that fin is frequently concealed and the threads are engaged blindly.

The present invention also aims to resolve this drawback.

Furthermore, sets of configurable elements exist making it possible to obtain different types of shoulder prostheses by assembling several elements from that line.

The existing lines are not, however, fully satisfactory, and the present invention also aims to resolve that drawback.

In a known manner, the concerned prosthesis comprises a humeral shaft and a proximal element capable of being mounted on said humeral shaft by mounting means.

According to the invention, said mounting means comprise:
- two conical cavities formed in a proximal end surface of the humeral shaft, partially intersecting one another, and at least one shim surface extending in the immediate vicinity of the openings of said cavities;
- a conical lug secured to said proximal element, the axis of which is offset in relation to a center of said proximal element, said lug being able to be engaged in one or the other of said conical cavities by jamming, and
- at the base of said lug, a polygonal portion centered on the axis of said lug, forming at least two shim facets capable of alternatingly coming, depending on the angular position of the proximal element in relation to the humeral shaft, into the immediate vicinity of said shim surface comprised by said humeral shaft.

In this way, the two conical cavities and said lug make it possible to mount the proximal element in two different positions, thereby making it possible to adjust the position of the humeral shaft in relation to the articular surface of the proximal element in the internal-external direction. Two useful adjustment positions are obtained owing to the fact that these two cavities partially intersect one another. Said shim surface and said shim facets jointly make up indexing means of the proximal element, making it possible, before the lug is jammed into one of the two conical cavities, to fix said proximal element in a predetermined angular position in relation to the humeral shaft, and therefore to adjust the position of said humeral shaft in relation to the articular surface of the proximal element in the front-back direction.

The invention thus allows a quick and easy choice, in peroperative, of the mounting of the proximal element in relation to the humeral shaft that allows perfect positioning of the humerus in relation to the scapula.

Preferably, said proximal end surface of the humeral shaft comprises an oblong recess in the bottom of which said conical cavities emerge, said recess being delimited by a wall of the humeral shaft partially forming said at least one shim surface.

The polygonal portion is thus engaged in said recess at the end of engagement of the lug in one or the other of said conical cavities, thereby bringing at least one shim facet formed by said polygonal portion into the immediate vicinity of the shim surface formed by said wall of the humeral shaft delimiting the recess.

Preferably, in that case, the recess is delimited, on two opposite sides, by two walls of the humeral shaft, perpendicular to the bottom of said recess, forming two shims surfaces as previously mentioned, opposite one another, and said polygonal portion forms at least two pairs of shim facets, the two facets of a same pair being symmetrical in relation to the axis of the lug and each being able to come into the vicinity of one of the two shim surfaces.

The shimming of said proximal element is thus done using the two shim surfaces and two corresponding shim facets, which ensures complete immobilization of the proximal element in relation to the humeral head.

Preferably, said polygonal portion is octagonal, thereby defining four pairs of parallel facets two by two, which allows eight distinct angular positions of said proximal element in relation to the humeral shaft.

Said proximal element can form a joint head with a convex articular surface, to make up a so-called "anatomical" shoulder prosthesis, or may include a cup forming a proximal cavity for receiving a joint part forming a concave articular surface, to make up a so-called "reverse" shoulder prosthesis.

Preferably, the outer side of the humeral shaft has two protruding side fins, one of which being situated on the front surface of the humeral shaft and the other of which being situated on the rear surface of that shaft.

These fins allow reinforced stabilization of the humeral shaft in the medullary channel of the humerus.

Advantageously, the humeral shaft comprises at least one hole formed in at least one fin, said hole being partially formed in the thickness of the shaft and partially in the fin, at least one recess with an inclined bottom wall being formed at the base of the fin across from said hole.

This hole is designed to receive at least one suture thread, allowing the reinsertion of fractured fragments of the humerus, in particular the reinsertion of tuberosities. Owing to its arrangement partially in the thickness of the shaft and partially in the fin, it has a large section and is therefore easy for the practitioner to find (this hole is generally not directly visible once the shaft is placed in the bone). Said inclined bottom wall of the recess forms a ramp guiding the suture needle toward that hole.

Advantageously, in that case, the humeral shaft has two of said recesses with an inclined bottom wall, formed on either side of the fin.

The guiding of the suture needle toward said hole is thus insured on both sides of the fin.

The set of elements according to the invention advantageously comprises:
- at least two humeral shafts with conical cavities and shim surfaces as previously mentioned, having different cervico-diaphyseal angles, for example 132° and 140°;
- at least two joint heads, each comprising a domed portion forming a convex articular surface and a lug capable of being inserted and jammed in either of the aforementioned conical cavities, the axis of the lug of one of said heads being offset in relation to the center of the dome portion of said head and the axis of the lug of the other head passing through the center of the dome portion of said other head;
- at least two cups each forming a proximal cavity for receiving a joint piece with a concave articular surface and comprising a lug that can be inserted and jammed in one or the other of the aforementioned conical cavities, the axis of the lug of one of said cups being offset relative to the center of said cup and the axis of the lug of the other cup passing through the center of said other cup.

This set of configurable elements makes it possible to form "anatomical" or "reverse" prostheses while making it possible to position the selected humeral shaft perfectly in relation to the articular surface connected to that humeral shaft.

The set may comprise a cup having a bottom perpendicular to the axis of the cavity formed by that cup, and another cup having a bottom not perpendicular to the axis of the cavity formed by said other cup.

The invention will be well understood, and other features and advantages thereof will appear, in reference to the appended diagrammatic drawing, showing, as a nonlimiting example, one preferred embodiment of various elements comprised by the concerned set of elements.
Figure 1 is a side view of a first type of shoulder prosthesis obtained by assembling certain elements from that line;
figure 2 is an enlarged perspective view of the metaphyseal portion of the humeral shaft comprised by said prosthesis;
figure 3 is a view of the proximal end of said humeral shaft, along the axis of conical cavities comprised by said metaphyseal portion;
figure 4 is a perspective view of that same metaphyseal portion and a joint head capable of being assembled to the humeral shaft;
figure 5 is a perspective view of the second type of shoulder prosthesis obtained by assembling some of the elements from the concerned set of elements;
figure 6 is an enlarged perspective view of the metaphyseal portion of the humeral shaft comprised by said prosthesis and a cup for assembling a joint part, capable of being fastened on the humeral head; and
figure 7 is a side view of the obtained shoulder prosthesis.

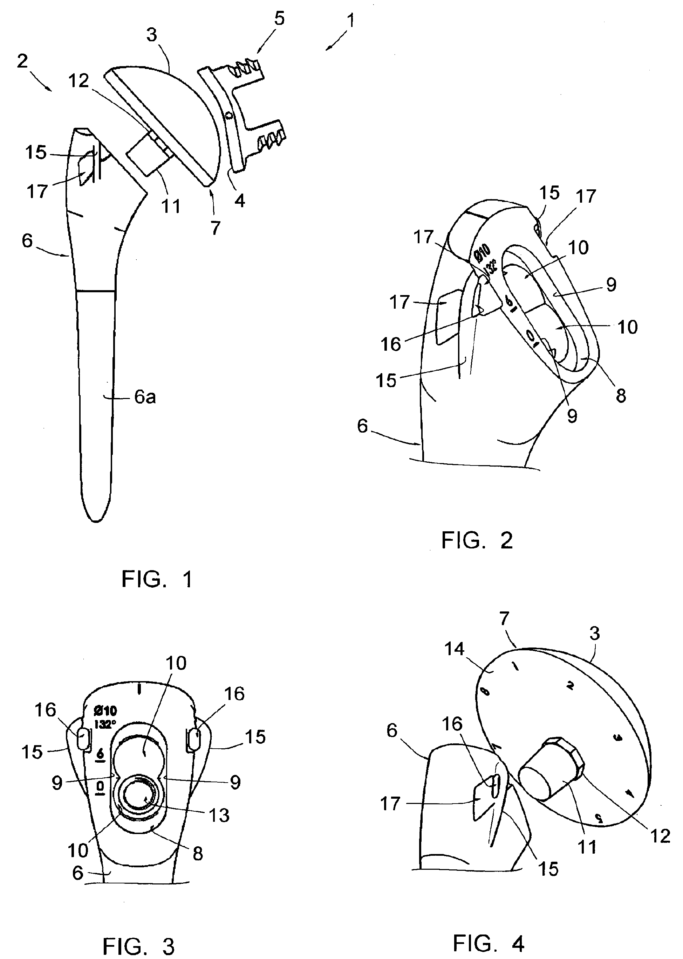

Figure 1 shows a shoulder joint prosthesis 1 of the so-called "anatomical" type, i.e. whereof the humeral portion 2 comprises a convex articular surface 3, cooperating with a concave articular surface 4 formed on a scapula implant 5.

The humeral portion 2 comprises a humeral shaft 6 and the proximal element, formed in that case by a joint head 7, said head 7 being able to be connected to the humeral shaft 6 by mounting means 8 to 12.

As shown more particularly by figures 1 to 4, these mounting means comprise an oblong recess 8 forming two surfaces 9, two conical cavities 10, a conical lug 11 and an octagonal portion 12.

The oblong recess 8 is formed in the humeral shaft 6, at an inclined proximal surface forming the end of said shaft 6. It comprises a bottom and is delimited by a peripheral wall perpendicular to that bottom forming said surfaces 9. The latter are opposite and parallel to one another.

The conical cavities 10 emerge in the bottom of the recess 8 and are formed such that their opening edges are in the immediate vicinity of the surfaces 9. They partially intersect one another, their axes in particular being able to be at a distance of 6 mm from one another.

A tapped hole 13 emerges in the bottom of one of the cavities 10 for mounting of the shaft 6 on a handling and impacting tool in the medullary channel of the humerus.

The conical lug 11 is secured to the dome portion 14 of the joint head 7 that forms said articular surface 3, and can be engaged by jamming in either of the conical cavities 10. Its axis is offset in relation to the center of the dome portion 14.

The octagonal portion 12 is formed at the base of the lug 11 and is centered on the axis of said lug. It forms four pairs of opposite shim facets two by two, capable of alternatingly coming, depending on the angular position of the head 7 in relation to the humeral shaft 6, into the immediate vicinity of the two shim surfaces 9.

As is understood in reference to figures 1 to 4, the two cavities 10 and the lug 11 make it possible to mount the head 7 in two distinct positions, thereby making it possible to adjust the position of the humeral shaft 6 in relation to the articular surface 3 in the internal-external direction. Two useful adjustment positions are obtained owing to the fact that these two cavities 10 partially intersect one another. The shim surfaces 9 and the shim facets formed by the octagonal portion 12 jointly make up means for indexing the head 7, making it possible, with jamming of the lug 11 in one of the two cavities 10, to fasten the head 7 in a predetermined angular position in relation to the humeral shaft 6, chosen from among eight possible angular positions, and therefore to adjust the position of said humeral shaft 6 in relation to the articular surface 3 in the front-back direction.

The set of elements according to the invention thus makes it possible to choose, easily and quickly in peroperative, the mounting of the head 7 in relation to the humeral shaft 6, which allows perfect positioning of the humerus in relation to the scapula.

The inclined proximal surface of the humeral shaft 6 bears marks, i.e. in the illustrated example (cf. figure 3): "Ø10" to indicate the diameter of the cavities 10, "132°" to indicate the cervico-diaphyseal angle, i.e. the angle between the axis of the diaphyseal portion 6a of the shaft 6 and the axis of the cavities 10, and "6" and "0" across from each of the cavities 10. The head 7 has (cf. figure 4), on the surface of its portion 14 including the lug 11, marks "1", "2", "3", "4", "5", "6", "7" and "8" identifying the eight angular positions allowed by the cooperation of said shim facets with the shim surfaces 9.

The external side of the humeral shaft 6 also bears two protruding side fins 15, one of which is situated on the front surface of the shaft 6 and the other of which is on the rear surface of said shaft. A hole 16 is formed through each fin 15, partially in the thickness of the shaft 6 and partially in the fin 15, as is particularly shown in figure 3. A recess 17 with an inclined bottom wall is formed at the base of the fin 15, across from said hole 16, on each side of the fin 15.

The fins 15, due to their positioning, allow reinforced stabilization of the humeral shaft 6 in the medullary channel of the humerus. The holes 16 are intended to receive suture threads, making it possible to reinsert fractured fragments of the humerus, in particular to reinsert tuberosities. Owing to their arrangement partially in the thickness of the shaft 6 and partially in the fin 15, they have a large section and are therefore easy for the practitioner to find (these holes are generally not directly visible once the shaft 6 is placed in the bone). The inclined bottom walls of the recesses 17 form ramps guiding a suture needle toward the holes 16.

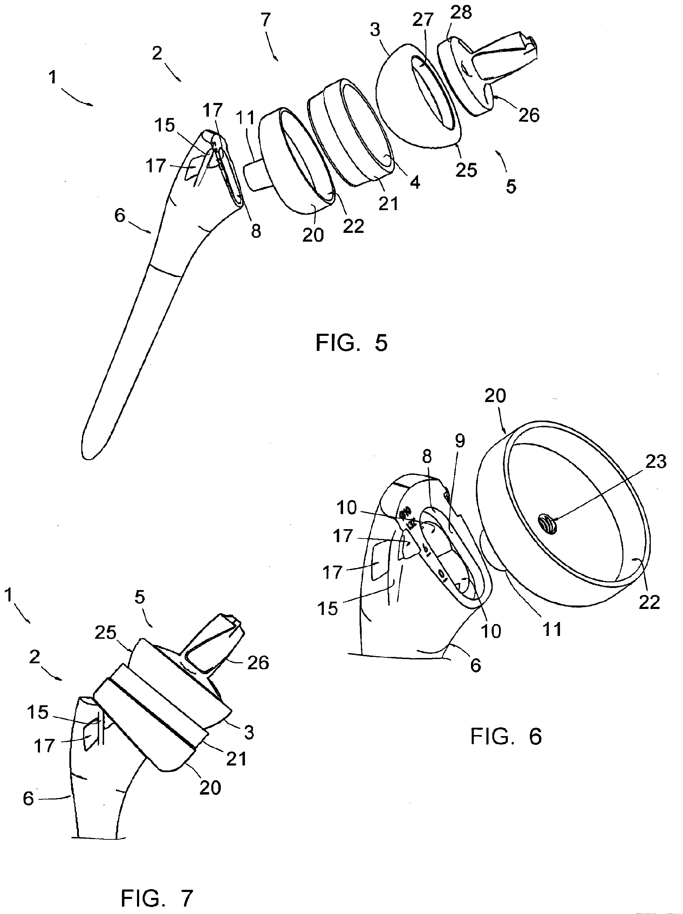

Figures 5 and 7 show a shoulder joint prosthesis 1 of the "reverse" type, i.e. whereof the humeral portion 2 comprises a concave articular surface 4, cooperating with a convex articular surface 3 formed on a scapula implant 5. For simplification, the elements or portions of elements already described that are found identically or similarly in this second type of prosthesis will be designated using the same numerical references and will not be described again.

In this case, the prosthesis 1 comprises a proximal element 7 formed from a cup 20 and a joint piece 21 forming the concave articular surface 4, to make up a so-called "reverse" shoulder prosthesis.

The cup 20 forms a cavity 22 for forcibly receiving the joint piece 21, and includes a lug 11 and an octagonal portion 12 as previously mentioned. It comprises a tapped hole 23 in its bottom, passing through said octagonal portion 12 and said lug 11, which allows a screw for extracting the lug 11 outside a cavity 10 to be engaged, said screw bearing against the bottom of said cavity 10.

The scapula implant 5, which, in the example of the "anatomical" prosthesis described above, was a single piece, is, in the "reverse" prosthesis, made up of a dome-shaped joint piece 25, forming the convex articular surface 3, and a base 26 for anchoring to the scapula. For mounting thereof on the base 26, the piece 25 comprises a cavity 27 in which a plate 28 comprised by the base 26 can be forcibly inserted.

The set of configurable elements according to the invention may comprise:
- at least two humeral shafts 6, having different cervico-diaphyseal angles, for example 132° and 140°;
- at least two joint heads 7, one of which comprises a lug 11 whereof the axis is offset in relation to the center of the dome portion 14 of said head, and the other of which comprises a lug 11 whereof the axis passes through the center of the dome portion 14 of said other head 7;
- at least two cups 20, one of which comprises a lug 11 whereof the axis is offset in relation to the center of that cup, and the other of which comprises a lug 11 whereof the axis passes through the center of said other cup;
- at least two additional cups 20, one of which has a bottom perpendicular to the axis of the cavity 22 formed by that cup, and the other of which has a bottom not perpendicular to the axis of the cavity 22 formed by that other cup.

The invention thus provides a shoulder joint prosthesis having the following decisive advantages:
- making it possible to choose, quickly and easily in peroperative, the mounting of the proximal element 7 in relation to the humeral shaft 6, which allows perfect positioning of the humerus in relation to the scapula;
- having reinforced stabilization in the medullary channel of the humerus owing to the fins 15;
- allowing easier placement of suture threads through the holes 16.

The invention also provides a set of configurable elements making it possible to form prostheses of the "anatomical" or "reverse" type while allowing perfect positioning of the selected humeral shaft 6 in relation to the articular surface connected to said humeral shaft.

The invention has been described above in reference to one embodiment provided as an example. It is of course not limited to that embodiment, but on the contrary encompasses all other embodiments covered by the appended claims.

## Claims

1. Shoulder joint prosthesis (1), comprising a humeral shaft (6) and a proximal element (7) capable of being mounted on said humeral shaft (6) by mounting means (8-12), said mounting means (8-12) comprising cavities (10) formed in a proximal end surface of the humeral shaft (6), partially intersecting one another, and a lug (11) secured to said proximal element (7);
**characterized in that**:
- said cavities (10) are two conical cavities (10), and at least one shim surface (9) extending in the immediate vicinity of the openings of said cavities (10);
- said lug (11) is conical and has an axis offset in relation to a center of said proximal element (7), said lug (11) being able to be engaged in one or the other of said conical cavities (10) by jamming, and
- at the base of said lug (11), a polygonal portion (12) centered on the axis of said lug, forming at least two shim facets capable of alternatingly coming, depending on the angular position of the proximal element (7) in relation to the humeral shaft (6), into the immediate vicinity of said shim surface (9) comprised by said humeral shaft (6).

2. Prosthesis (1) according to claim 1, **characterized in that** said proximal end surface of the humeral shaft (6) comprises an oblong recess (8) in the bottom of which said conical cavities (10) emerge, said recess (8) being delimited by a wall of the humeral shaft (6) partially forming said at least one shim surface (9).

3. Prosthesis (1) according to claim 2, **characterized in that** said recess (8) is delimited, on two opposite sides, by two walls of the humeral shaft (6), perpendicular to the bottom of said recess (8), forming two shims surfaces (9) as previously mentioned, opposite one another, and said polygonal portion (12) forms at least two pairs of shim facets, the two facets of a same pair being symmetrical in relation to the axis of the lug (11) and each being able to come into the vicinity of one of the two shim surfaces (9).

4. Prosthesis (1) according to claim 3, **characterized in that** said polygonal portion (12) is octagonal, thereby defining four pairs of parallel facets two by two, which allows eight distinct angular positions of said proximal element in relation to the humeral shaft (6).

5. Prosthesis (1) according to anyone of claims 1-4, **characterized in that** said proximal element forms a joint head (7) with a convex articular surface (3).

6. Prosthesis (1) according to anyone of claims 1-4, **characterized in that** said proximal element includes a cup (20) forming a proximal cavity (22) for receiving a joint part (21) forming a concave articular surface (4).

7. Prosthesis (1) according to anyone of claims 1-6, **characterized in that** the outer side of the humeral shaft (6) has two protruding side fins (15), one of which being situated on the front surface of the humeral shaft (6) and the other of which being situated on the rear surface of that shaft.

8. Prosthesis (1) according to claim 7, **characterized in that** the humeral shaft (6) comprises at least one hole (16) formed in at least one fin (15), said hole (16) being partially formed in the thickness of the shaft (6) and partially in the fin (15), at least one recess (17) with an inclined bottom wall being formed at the base of the fin (15) across from said hole (16).

9. Prosthesis (1) according to claim 8, **characterized in that** the humeral shaft (6) has two of said recesses (17), formed on either side of the fin (15).

10. Set of configurable elements making it possible to form the prosthesis (1) according to anyone of claims 1-9, **characterized in that** comprises:
- at least two humeral shafts (6) with conical cavities (10) and shim surfaces (9) as previously mentioned, having different cervico-diaphyseal angles, for example 132° and 140°;
- at least two joint heads (7), each comprising a domed portion (14) forming a convex articular surface (3) and a lug (11) capable of being inserted and jammed in either of said conical cavities (10), the axis of the lug (11) of one of said heads being offset in relation to the center of the dome portion (14) of said head and the axis of the lug (11) of the other head passing through the center of the dome portion (14) of said other head;
- at least two cups (20) each forming a proximal cavity (22) for receiving a joint piece (21) with a concave articular surface (4) and comprising a lug (11) that can be inserted and jammed in one or the other of the aforementioned conical cavities (10), the axis of the lug (11) of one of said cups (20) being offset relative to the center of said cup and the axis of the lug (11) of the other cup (20) passing through the center of said other cup.

11. Set according to claim 10, **characterized in that** it comprises a cup (20) having a bottom perpendicular to the axis of the cavity (22) formed by that cup, and another cup (20) having a bottom not perpendicular to the axis of the cavity (22) formed by said other cup.

## Patentansprüche

1. Schultergelenkprothese (1), umfassend einen Oberarmknochenschaft (6) und ein proximales Element (7), das in der Lage ist, an dem Oberarmknochenschaft (6) mit Befestigungsmitteln (8 bis 12) befestigt zu werden, wobei die Befestigungsmittel (8 bis 12) Hohlräume (10) umfassen, die in einer proximalen Endfläche des Oberarmknochenschafts (6) ausgebildet sind und sich teilweise schneiden, sowie einen Ansatz (11), der an dem proximalen Element (7) gesichert ist;
**dadurch gekennzeichnet, dass**:
- die Hohlräume (10) zwei konische Hohlräume (10) sind, und mindestens eine Einstellfläche (9) in der unmittelbaren Nähe der Öffnungen der Hohlräume (10) verläuft;
- der Ansatz (11) konisch ist und eine Achse versetzt zu einer Mitte des proximalen Elements (7) aufweist, wobei der Ansatz (11) durch Verkeilen in den einen oder den anderen der konischen Hohlräume (10) einsteckbar ist, und
- am Fuß des Ansatzes (11) ein vieleckiger Abschnitt (12) auf der Achse des Ansatzes zentriert ist und so mindestens zwei Einstell-Seitenflächen bildet, die geeignet sind, je nach Winkellage des proximalen Elements (7) bezogen auf den Oberarmknochenschaft (6) abwechselnd in die unmittelbare Nähe der Einstellfläche (9) zu gelangen, die der Oberarmknochenschaft (6) umfasst.

2. Prothese (1) nach Anspruch 1, **dadurch gekennzeichnet, dass** die proximale Endfläche des Oberarmknochenschafts (6) eine längliche Vertiefung (8) umfasst, in deren Boden die konischen Hohlräume (10) eingelassen sind, wobei die Vertiefung (8) von einer Wand des Oberarmknochenschafts (6) begrenzt ist, die teilweise die mindestens eine Einstellfläche (9) bildet.

3. Prothese (1) nach Anspruch 2, **dadurch gekennzeichnet, dass** die Vertiefung (8), auf zwei gegenüberliegenden Seiten, von zwei Wänden des Oberarmknochenschafts (6) senkrecht zum Boden der Vertiefung (8) begrenzt ist, die zwei wie zuvor erwähnte einander gegenüberliegende Einstellflächen (9) bilden, und der vieleckige Abschnitt (12) mindestens zwei Paar von Einstell-Seitenflächen bildet, wobei die beiden Seitenflächen von ein und demselben Paar bezogen auf die Achse des Ansatzes (11) symmetrisch sind und jeweils in der Lage sind, in die Nähe von einer der beiden Einstellflächen (9) zu gelangen.

4. Prothese (1) nach Anspruch 3, **dadurch gekennzeichnet, dass** der vieleckige Abschnitt (12) achteckig ist, wodurch vier Paar von jeweils zwei zueinander parallelen Seitenflächen definiert sind, woraus sich acht unterschiedliche Winkellagen des proximalen Elements bezogen auf den Oberarmknochenschaft (6) ergeben.

5. Prothese (1) nach einem der Ansprüche 1 bis 4, **dadurch gekennzeichnet, dass** das proximale Element einen Gelenkkopf (7) mit einer konvexen Gelenkfläche (3) bildet.

6. Prothese (1) nach einem der Ansprüche 1 bis 4, **dadurch gekennzeichnet, dass** das proximale Element eine Pfanne (20) aufweist, die einen proximalen Hohlraum (22) zum Aufnehmen eines Gelenkteils (21) bildet, der eine konkave Gelenkfläche (4) bildet.

7. Prothese (1) nach einem der Ansprüche 1 bis 6, **dadurch gekennzeichnet, dass** die Außenseite des Oberarmknochenschafts (6) zwei vorstehende Seitenrippen (15) aufweist, von denen sich eine an der Vorderseite des Oberarmknochenschafts (6) befindet und sich die andere an der Rückseite dieses Schafts befindet.

8. Prothese (1) nach Anspruch 7, **dadurch gekennzeichnet, dass** der Oberarmknochenschaft (6) mindestens ein Loch (16) umfasst, das in mindestens einer Rippe (15) ausgebildet ist, wobei das Loch (16) teilweise in der Dicke des Schafts (6) und teilweise in der Rippe (15) ausgebildet ist, wobei mindestens eine Vertiefung (17) mit einer geneigten Bodenwand an der Grundfläche der Rippe (15) auf der anderen Seite von dem Loch (16) ausgebildet ist.

9. Prothese (1) nach Anspruch 8, **dadurch gekennzeichnet, dass** der Oberarmknochenschaft (6) zwei der Vertiefungen (17), auf jeder Seite der Rippe (15) ausgebildet, aufweist.

10. Satz aus konfigurierbaren Elementen, mit denen eine Herstellung der Prothese (1) nach einem der Ansprüche 1 bis 9 möglich ist, **dadurch gekennzeichnet, dass** er Folgendes umfasst:
- mindestens zwei Oberarmknochenschäfte (6) mit wie zuvor erwähnten konischen Hohlräumen (10) und Einstellflächen (9), mit unterschiedlichen Hals-Schaft-Winkeln, beispielsweise 132° und 140°;
- mindestens zwei Gelenkköpfe (7), von denen jeder einen gewölbten Abschnitt (14), der eine konvexe Gelenkfläche (3) bildet, und einen Ansatz (11) umfasst, der in einen beliebigen der konischen Hohlräume (10) einsetzbar und verkeilbar ist, wobei die Achse des Ansatzes (11) von einem der Köpfe bezogen auf die Mitte des Wölbungsabschnitts (14) des Kopfs versetzt ist und die Achse des Ansatzes (11) des anderen Kopfs durch die Mitte des Wölbungsabschnitts (14) des anderen Kopfs verläuft;
- mindestens zwei Pfannen (20), die jeweils einen proximalen Hohlraum (22) zum Aufnehmen eines Gelenkteils (21) mit einer konkaven Gelenkfläche (4) bilden und einen Ansatz (11) umfassen, der in den einen oder den anderen der zuvor erwähnten konischen Hohlräume (10) eingesetzt und verkeilt werden kann, wobei die Achse des Ansatzes (11) von einer der Pfannen (20) bezogen auf die Mitte der Pfanne versetzt ist und die Achse des Ansatzes (11) der anderen Pfanne (20) durch die Mitte der anderen Pfanne verläuft.

11. Satz nach Anspruch 10, **dadurch gekennzeichnet, dass** er eine Pfanne (20) mit einem Boden senkrecht zur Achse des Hohlraums (22) umfasst, der von der Pfanne gebildet wird, und eine weitere Pfanne (20) mit einem Boden, der nicht senkrecht zur Achse des Hohlraums (22) verläuft, der von der weiteren Pfanne gebildet wird.

## Revendications

1. Prothèse (1) d'articulation de l'épaule, comprenant une tige humérale (6) et un élément proximal (7) apte à être monté sur cette tige humérale (6) par des moyens de montage (8 à12), lesdits moyens de montage (8-12) comprenant des cavités (10) aménagées dans une face proximale d'extrémité de la tige humérale (6), en intersection partielle l'une avec l'autre, et un plot (11) solidaire dudit élément proximal (7) ;
**caractérisée en ce que** :
- lesdites cavités coniques (10) sont deux cavités coniques (10), et au moins une face de calage (9) s'étend à proximité immédiate des ouvertures de ces cavités (10) ;
- ledit plot (11) est conique et a un axe est décalé par rapport à un centre dudit élément proximal (7), ce plot (11) étant apte à être engagé avec coincement dans l'une ou l'autre desdites cavités coniques (10), et
- à la base de ce plot (11), une portion polygonale (12) centrée sur l'axe de ce plot, formant au moins deux facettes de calage aptes à venir alternativement l'une à l'autre, selon la position angulaire de l'élément proximal (7) par rapport à la tige humérale (6), à proximité immédiate de ladite face de calage (9) que comprend la tige humérale (6).

2. Prothèse (1) selon la revendication 1, **caractérisée en ce que** ladite face proximale d'extrémité de la tige humérale (6) comprend un évidement oblong (8) dans le fond duquel débouchent lesdites cavités coniques (10), cet évidement (8) étant délimité par une paroi de la tige humérale (6) formant en partie ladite au moins une face de calage (9).

3. Prothèse (1) selon la revendication 2, **caractérisée en ce que** ledit évidement (8) est délimité, sur deux côtés opposés, par deux parois de la tige humérale (6), perpendiculaires au fond de cet évidement (8), formant deux faces de calage (9) telles que précitées, opposées l'une à l'autre, et **en ce que** ladite portion polygonale (12) forme au moins deux paires de facettes de calage, les deux facettes d'une même paire étant symétriques par rapport à l'axe du plot (11) et étant chacune apte à venir à proximité de l'une des deux faces de calage (9).

4. Prothèse (1) selon la revendication 3, **caractérisée en ce que** ladite portion polygonale (12) est de forme octogonale, définissant quatre paires de facettes parallèles deux à deux, autorisant huit positions angulaires distinctes dudit élément proximal (7) par rapport à la tige humérale (6).

5. Prothèse (1) selon l'une des revendications 1 à 4, **caractérisée en ce que** ledit élément proximal forme une tête d'articulation (7) à surface articulaire convexe (3).

6. Prothèse (1) selon l'une des revendications 1 à 4, **caractérisée en ce que** ledit élément proximal (7) inclut une cupule (20) formant une cavité proximale (22) de réception d'une pièce articulaire (21) formant une surface d'articulation concave (4).

7. Prothèse (1) selon l'une des revendications 1 à 6, **caractérisée en ce que** la tige humérale (6) présente, sur son côté externe, deux ailerons latéraux saillants (15), dont un est situé sur la face antérieure de la tige humérale (6) et l'autre sur la face postérieure de cette tige.

8. Prothèse (1) selon la revendication 7, **caractérisée en ce que** la tige humérale (6) comprend au moins un trou (16) aménagé dans au moins un aileron (15), ce trou (16) étant aménagé pour partie dans l'épaisseur de la tige (6) et pour partie dans l'aileron (15), au moins un évidement (17) à paroi de fond inclinée étant aménagé à la base de l'aileron (15) en regard de ce trou (16).

9. Prothèse (1) selon la revendication 8, **caractérisée en ce que** la tige humérale (6) présente deux évidements (17), aménagés de part et d'autre de l'aileron (15).

10. Gamme d'éléments modulaires permettant la constitution de la prothèse (1) selon l'une quelconque des revendications 1 à 9, **caractérisée en ce qu'**elle comprend :
- au moins deux tiges humérales (6) à cavités coniques (10) et faces de calage (9) telles que précitées, ayant des angles cervico-diaphysaires différents, par exemple de 132° et de 140° ;
- au moins deux têtes d'articulation (7), comprenant chacune une partie en dôme (14) formant une surface articulaire convexe (3) et un plot (11) apte à être inséré et coincé dans l'une ou l'autre des cavités coniques (10) précitées, le plot (11) de l'une de ces têtes ayant son axe décalé par rapport au centre de la partie en dôme (14) de cette tête et le plot (11) de l'autre tête ayant son axe passant par le centre de la partie en dôme (14) de cette autre tête ;
- au moins deux cupules (20) formant chacune une cavité proximale (22) de réception d'une pièce articulaire (21) à surface articulaire concave (4) et comprenant un plot (11) apte à être inséré et coincé dans l'une ou l'autre des cavités coniques (10) précitées, le plot (11) de l'une de ces cupules (20) ayant son axe décalé par rapport au centre de cette cupule et le plot (11) de l'autre cupule (20) ayant son axe passant par le centre de cette autre cupule.

11. Gamme d'éléments modulaires selon la revendication 10, **caractérisée en ce qu'**elle comprend une cupule (20) présentant un fond perpendiculaire à l'axe de la cavité (22) que forme cette cupule, et une autre cupule (20) présentant un fond non perpendiculaire à l'axe de la cavité (22) que forme cette autre cupule.
